# EUROPÄISCHE PATENTSCHRIFT

(11) **EP 2 845 554 B1**
(45) Veröffentlichungstag und Bekanntmachung des Hinweises auf die Patenterteilung: **07.06.2017**
(21) Anmeldenummer: 14184046.2
(22) Anmeldetag: 09.09.2014
(51) Int. Cl.: A61B 17/88, A61B 17/70, A61B 17/86, A61B 17/00, B01F 13/00, B01F 15/02, B01F 5/06

(54) **Medizinisches Instrument**
Medical instrument
Instrument médical

(30) Priorität: 10.09.2013 DE 102013109895
(43) Veröffentlichungstag der Anmeldung: 11.03.2015
(73) Patentinhaber: AESCULAP AG, 78532 Tuttlingen (DE)
(72) Erfinder: Hoefer, Fabian, 78532 Tuttlingen (DE); Krüger, Sven, 78647 Trossingen (DE)
(74) Vertreter: Hoeger, Stellrecht & Partner Patentanwälte mbB

(56) Entgegenhaltungen:
- EP-A1- 0 747 114
- DE-A1-102010 016 812
- DE-U1-202008 006 870
- US-A1- 2009 264 895

## Beschreibung

Die vorliegende Erfindung betrifft ein medizinisches Instrument umfassend einen Werkzeugabschnitt mit einem ersten Werkzeugelement zum in Eingriff Bringen mit einem korrespondierenden zweiten Werkzeugelement an einem Implantat und mit einem am oder im Instrument angeordneten oder ausgebildeten Knochenzementbehälter.

Medizinische Instrumente der eingangs beschriebenen Art sind in vielfältiger Form bekannt. Beispielsweise werden medizinische Instrumente in Form von Schraubendrehern verwendet, um Schrauben, insbesondere Pedikelschrauben, von Wirbelsäulenstabilisierungssystemen in Wirbel einer menschlichen oder tierischen Wirbelsäule einzuschrauben. Es ist bekannt, derartige Schrauben mit einem Zementkanal auszubilden, also insbesondere in kannulierter Form, so dass nach dem Einbringen der Schrauben in einen Knochen oder ein Knochenteil, beispielsweise in einen Pedikel eines Wirbels, nach der Implantation der Schraube zusätzlich noch Knochenzement durch den Zementkanal der Schraube appliziert werden kann, um eine noch bessere Verankerung der Knochenschraube zu erreichen.

Aus der US 2009/0264895 A1 sind Systeme und Verfahren zum Implantieren von Knochenbefestigungselementen und zum Ausbringen von Knochenfüllmaterial bekannt. In der DE 10 2010 016 812 A1 sind Knochenschrauben beschrieben. Die EP 0 747 114 A1 offenbart Packungen mit anwendungsfertigem flüssigem Knochenzement.

Es ist daher eine Aufgabe der vorliegenden Erfindung, ein medizinisches Instrument der eingangs beschriebenen Art so zu verbessern, dass Implantate, insbesondere Knochenschrauben, einfach und schnell implantiert werden können.

Diese Aufgabe wird bei einem medizinischen Instrument der eingangs beschriebenen Art erfindungsgemäß dadurch gelöst, dass das Instrument insgesamt einstückig ausgebildet ist.

Der Knochenzementbehälter ermöglicht es, optional Knochenzement, ein beliebiges Fluid, ein Pulver und/oder ein Augmentat bereits im Instrument zu lagern. Damit ist es möglich, mit dem Instrument zunächst ein Implantat einzubringen, und zwar durch Koppeln des ersten Werkzeugelements des Instruments mit dem korrespondierenden zweiten Werkzeugelement des Implantats. Beispielsweise bei einem Implantat in Form einer Knochenschraube kann so die Knochenschraube in ein Knochenteil, beispielsweise einen Wirbel, eingeschraubt werden. Wenn das Implantat seine endgültige Position erreicht hat, kann dann in einem zweiten Schritt mit dem Instrument optional Knochenzement direkt appliziert werden, ohne das Instrument wieder vom Implantat entfernen zu müssen, was bei herkömmlichen medizinischen Instrumenten der Fall ist. Die Applikation des Knochenzements erfolgt herkömmlicherweise in einem dritten Schritt nach Entfernen des Einschraubinstruments vom und Ankoppeln beispielsweise einer Kanüle mit Knochenzement an das Implantat und anschließendes Applizieren des Knochenzements durch das Implantat hindurch. Das erfindungsgemäß weitergebildete medizinische Instrument gestattet so insbesondere eine deutlich vereinfachte Handhabung des Knochenzements. Zum Beispiel ist es so möglich, das zum Einbringen des Implantats vorgesehene Instrument mit genau der richtigen Menge an Knochenzement zu beschicken, die zum Zementieren des Implantats erforderlich ist. Ferner ist kein externes Mischen des Knochenzements mehr notwendig, sodass eine Verschmutzung und Kontamination des Wundbereichs vermieden werden können. Ferner kann auch eine Verschmutzung des Implantats im Kopplungsbereich vermieden werden. Wird das Instrument als Einweginstrument ausgebildet, ist zudem eine einfache Entsorgung des Instruments und des Knochenzementbehälters möglich. Wie bereits erwähnt kann das Instrument auch mit Fluiden, insbesondere Spülfluiden, oder Pulvern befüllt werden oder befüllt sein, und die Applikation des im Knochenzementbehälters enthaltenen Materials vereinfachen, da ein Implantat eingebracht und anschließend, ohne Instrumentenwechsel, der Inhalt des Knochenzementbehälters appliziert werden kann. Die Herstellung des Instruments lässt sich dadurch besonders einfach gestalten, dass es insgesamt einstückig ausgebildet ist. Beispielsweise kann es durch Spritzgießen geformt sein.

Besonders einfach und kompakt ausbilden lässt sich das Instrument, wenn es mindestens einen hülsenförmigen Abschnitt aufweist, und wenn der Knochenzementbehälter im Bereich des hülsenförmigen Abschnitts angeordnet oder ausgebildet ist. Knochenzement kann so direkt im hülsenförmigen Abschnitt untergebracht werden. Vorzugsweise wird eine Wandstärke des hülsenförmigen Abschnitts so gewählt, dass die für das Einbringen des Implantats ausreichende Stabilität des Instruments gewährleistet ist.

Vorzugsweise bildet der hülsenförmige Abschnitt den Knochenzementbehälter oder bildet ihn im Wesentlichen. Auf diese Weise werden keine zusätzlichen Elemente am Instrument benötigt, um den Knochenzement aufzunehmen. Beispielsweise kann am Instrument statt eines massiven Schafts ein hülsenförmiger Schaft vorgesehen sein, in welchem Knochenzement beispielsweise direkt oder in einer darin eingesetzten Hülse oder Hülle gelagert werden kann.

Gemäß einer bevorzugten Ausführungsform der Erfindung kann vorgesehen sein, dass der Knochenzementbehälter mindestens eine erste Kammer und mindestens eine zweite Kammer umfasst und dass die mindestens eine erste und die mindestens eine zweite Kammer durch mindestens ein Trennelement räumlich voneinander getrennt sind. Der Knochenzementbehälter kann beispielsweise zwei, drei oder vier Kammern umfassen. Insbesondere für Knochenzement, welcher durch Mischen von zwei Komponenten gebildet wird, umfasst der Knochenzementbehälter vorzugsweise zwei Kammern. Durch das mindestens eine Trennelement können somit die in den Kammern des Knochenzementbehälters enthaltenen Knochenzementkomponenten erst dann miteinander in Kontakt treten, wenn dies gewünscht ist. Beispielsweise kann zu diesem Zweck das Trennelement zerstört oder entfernt werden, um ein Vermischen der Komponenten des Knochenzements gezielt zu ermöglichen. Günstig ist es, wenn das mindestens eine Trennelement in Form einer Trennwand ausgebildet ist, die sich quer oder parallel zu einer Längsachse des Knochenzementbehälters erstreckt. Beispielsweise können zwei Kammern des Knochenzementbehälters in Längsrichtung hintereinander angeordnet und durch ein sich quer zur Längsachse erstreckendes Trennelement getrennt sein. Alternativ ist auch denkbar, dass sich zwei oder mehr Kammern in Längsrichtung des Knochenzementbehälters erstrecken, wobei diese dann durch sich ebenfalls in Längsrichtung erstreckenden Trennwände voneinander getrennt sein können. Alternativ ist es auch denkbar, die Komponenten des Knochenzements in separaten Behältnissen im Knochenzementbehälter unterzubringen, beispielsweise in dünnwandigen Folienverpackungen. Diese können dann beispielsweise durchstochen werden, um ein Vermischen der Komponenten zu ermöglichen. Insbesondere können Sie aus den separaten Behältnissen in einem Mischraum oder Mischbereich zusammengebracht werden.

Vorteilhaft ist es, wenn das Instrument eine Vorschubeinrichtung zum Durchmischen und/oder Ausbringen von im Knochenzementbehälter befindlichem Knochenzement umfasst. Insbesondere kann die Vorschubeinrichtung ausgebildet sein zum Vorschieben des Knochenzements in distaler Richtung. Mit der Vorschubeinrichtung lässt sich Knochenzement auf einfache und sichere Weise durch einen Zementkanal des Implantats hindurch applizieren.

Besonders einfach wird der Aufbau der Vorschubeinrichtung, wenn diese einen Stößel umfasst mit einem an einen inneren Querschnitt des Knochenzementbehälters angepassten Vorschubglied. Beispielsweise kann das Vorschubglied in Form eines kleinen Kolbens ausgebildet sein, welcher optional ein umlaufendes Dichtelement umfasst, um den Knochenzement aus dem vorzugsweise hohlzylindrischen Knochenzementbehälter heraus zu drücken. Ferner kann der Stößel auch zur feinfühligeren Kraftübertragung und damit zur genaueren Applikation des Inhalts des Knochenzementbehälters, beispielsweise Knochenzement oder ein Fluid, verwendet werden.

Um das Instrument besonders einfach Handhaben zu können, ist es vorteilhaft, wenn die Vorschubeinrichtung ein Betätigungselement umfasst, welches ein proximales Ende der Vorschubeinrichtung definiert und mit dem Stößel temporär gekoppelt oder einstückig mit diesem ausgebildet ist. Beispielsweise kann das Betätigungselement proximalseitig aus dem Instrument vorstehen, so dass auf es eine Kraft in distaler Richtung ausgeübt werden kann, um Knochenzement aus dem Knochenzementbehälter in distaler Richtung aus dem Instrument auszutreiben.

Vorzugsweise umfasst das erste Werkzeugelement einen Knochenzementkanal, welcher das erste Werkzeugelement durchsetzt und mit dem Knochenzementbehälter in Fluidverbindung steht. So ist es möglich, Knochenzement direkt durch das erste Werkzeugelement hindurch in das Implantat zu treiben, insbesondere auch dann, wenn das Instrument mit seinem ersten Werkzeugelement mit dem Implantat gekoppelt ist.

Günstig ist es, wenn das Instrument ein Verschlusselement umfasst, welches den Knochenzementkanal und/oder den Knochenzementbehälter zum Knochenzementkanal hin verschließt. Das Verschlusselement verhindert somit insbesondere, dass Knochenzement durch den Knochenzementkanal hindurch austreten kann. Das Verschlusselement kann auch den Knochenzementbehälter zu einem Mischbereich oder -raum hin verschließen.

Günstigerweise ist das Verschlusselement irreversibel ablösbar oder durchtrennbar zum Freigeben des Knochenzementkanals oder des Knochenzementbehälters. Der im Knochenzementbehälter untergebrachte Knochenzement und/oder dessen getrennt gelagerte Komponenten können so nach Ablösen oder Durchtrennen des Verschlusselements durch den Knochenzementkanal hindurch ausgebracht werden.

Besonders gezielt und sauber lässt sich Knochenzement in das Implantat einbringen, wenn das Instrument einen Zementauslassstutzen aufweist. Dieser kann vorzugsweise distalseitig über das erste Werkzeugelement vorstehend ausgebildet sein. So kann verhindert werden, dass Knochenzement im Bereich des ersten Werkzeugelements austreten und dieses verschmutzen kann.

Vorteilhaft ist es, wenn sich der Zementkanal durch den Zementauslassstutzen hindurch erstreckt. So kann auf einfache Weise Knochenzement in distaler Richtung aus dem Instrument in das Implantat eingebracht werden.

Gemäß einer weiteren bevorzugten Ausführungsform des medizinischen Instruments kann vorgesehen sein, dass der Knochenzementbehälter mindestens einen Mischbereich umfasst, welcher mindestens einen, vorzugsweise eine Mehrzahl von Mischelementen umfasst. Im Mischbereich, der insbesondere in Form eines Mischraums ausgebildet sein kann, kann aus zwei oder mehr Komponenten gebildeter Knochenzement vermischt werden. Beispielsweise zweikomponentiger Knochenzement, welcher einen Binder und einen Härter umfasst, kann im Mischbereich optimal vermischt werden, um die gewünschte chemische Reaktion zwischen den Komponenten des Knochenzements starten zu können.

Das Mischen von zwei oder mehrkomponentigem Knochenzement kann auf einfache Weise dadurch verbessert werden, dass die Mehrzahl von Mischelementen in Form von Mischvorsprüngen ausgebildet ist, die von einer inneren Wandfläche des Knochenzementbehälters vorstehend ausgebildet sind. Beispielsweise können diese flügelartig ausgebildet sein, so dass beim Vorschieben des Knochenzements dieser im Knochenzementbehälter nicht laminar, sondern quasi turbulent strömt, wodurch eine Vermischung der Komponenten des Knochenzements signifikant verbessert wird.

Vorteilhaft ist es, wenn der Knochenzementbehälter Knochenzement enthält. Mit anderen Worten ist dann das Instrument nicht nur vorbereitet, Knochenzement im Knochenzementbehälter aufzunehmen, sondern enthält bereits Knochenzement, der nach dem Einbringen des Implantats direkt aus dem Knochenzementbehälter heraus appliziert werden kann, ohne das Instrument entfernen und dann das Implantat mit einem Knochenzementapplikator koppeln zu müssen. Das Instrument bildet auf diese Weise somit gleichzeitig auch den Zementapplikator.

Günstig ist es, wenn der Knochenzement mindestens eine erste Knochenzementkomponente und mindestens eine zweite Knochenzementkomponente umfasst, die räumlich voneinander getrennt im Knochenzementbehälter enthalten sind. Dies ermöglicht es, den Knochenzement im Instrument auch längerfristig zu lagern, ohne dass die Aushärtereaktion vor der eigentlichen Applikation des Knochenzements einsetzen kann.

Vorzugsweise enthält die mindestens eine erste Kammer die mindestens eine erste Knochenzementkomponente und enthält die mindestens eine zweite Kammer die mindestens eine zweite Knochenzementkomponente. So können die Knochenzementkomponenten einfach und sicher getrennt in den dafür vorgesehenen Kammern des Knochenzementbehälters gelagert werden, und zwar ohne dass sie in unerwünschter Weise vor der Applikation des Knochenzements miteinander reagieren können.

Zur einfachen und sicheren Handhabung des Instruments ist es günstig, wenn es ein Griffelement umfasst, welches ein proximales Ende des Instruments bildet oder im Bereich eines proximalen Endes des Instruments angeordnet oder ausgebildet ist. So lässt es sich einfach und sicher, insbesondere mit seinem distalen Ende, an ein Implantat koppeln, um dieses in ein Knochenteil einzubringen, und danach direkt Knochenzement zu applizieren.

Besonders kompakt ausbilden lässt sich das Instrument, wenn das Griffelement den Knochenzementbehälter vollständig oder teilweise umfasst. Es kann also Knochenzement quasi im Inneren des Griffelements untergebracht werden.

Um das Instrument teilweise wiederverwenden zu können, ist es vorteilhaft, wenn das Griffelement und der Knochenzementbehälter lösbar verbindbar sind. Nach Abschluss der Implantation des Implantats und nach der Applikation des Knochenzements kann so beispielsweise das Griffelement vom Knochenzementbehälter entfernt, für die nächste Implantation gereinigt und dann wieder mit einem neuen Knochenzementbehälter verbunden werden.

Gemäß einer weiteren bevorzugten Ausführungsform der Erfindung kann vorgesehen sein, dass das erste Werkzeugelement in Form eines Werkzeugvorsprungs ausgebildet ist zum in Eingriff Bringen mit einer korrespondierend ausgebildeten Werkzeugaufnahme an einem Implantat. So kann das Instrument zur Übertragung eines Drehmoments auf das Implantat ausgebildet sowie einfach und sicher an das Implantat angekoppelt werden.

Auf einfache Weise lassen sich Drehmomente sicher auf ein Implantat übertragen, wenn das erste Werkzeugelement in Form eines Mehrkants, eines Vielrunds oder eines Gewindeabschnitts ausgebildet ist. So kann das Instrument insbesondere in eine korrespondierende Werkzeugaufnahme gesteckt oder eingeschraubt werden.

Kostengünstig und einfach herstellen lässt sich das Instrument, wenn es vollständig oder teilweise aus einem oder mehreren Kunststoffen gebildet ist. Beispielsweise kann es durch Spritzgießen hergestellt sein. Insbesondere können unterschiedliche Kunststoffe verwendet werden zur Ausbildung des ersten Werkzeugelements und des Knochenzementbehälters. Beispielsweise können zur Ausbildung des ersten Werkzeugelements Kunststoffe verwendet werden, die nur wenig verformbar, also relativ hart sind. Für den Knochenzementbehälter können vorzugsweise transparente oder zumindest transluzente Kunststoffe verwendet werden, damit ein Operateur direkt erkennen kann, wieviel Knochenzement noch im Knochenzementbehälter ist.

Zum Einschrauben eines Implantats, insbesondere einer Knochenschraube, ist es vorteilhaft, wenn das Instrument in Form eines Schraubendrehers ausgebildet ist.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines medizinischen Instruments beim Einbringen eines Implantats in ein Knochenteil;
- Figur 2:: eine ausschnittsweise Längsschnittansicht eines mit einem Implantat gekoppelten medizinischen Instruments nach dem Einbringen des Implantats in das Knochenteil;
- Figur 3:: eine Schnittansicht eines medizinischen Instruments mit einer ersten Variante eines Knochenzementbehälters; und
- Figur 4:: eine Schnittansicht ähnlich Figur 3 eines weiteren Ausführungsbeispiels eines medizinischen Instruments mit einem Mischbereich.

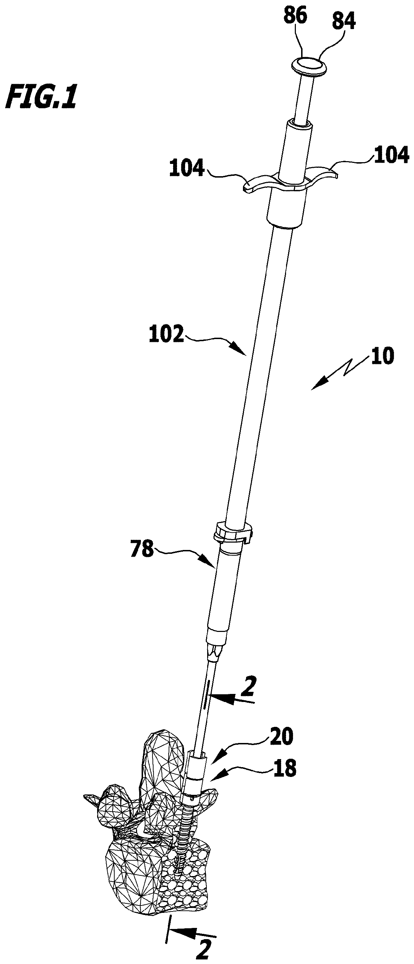

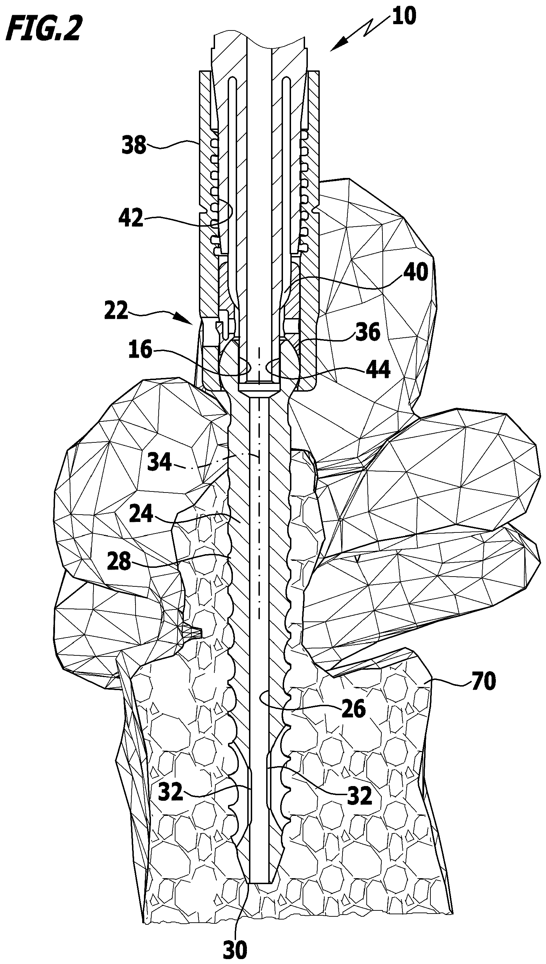

In Figur 1 ist ein medizinisches Instrument schematisch dargestellt und mit dem Bezugszeichen 10 bezeichnet. Es umfasst einen Werkzeugabschnitt 12 mit einem ersten Werkzeugelement 66 zum in Eingriff Bringen mit einem zweiten Werkzeugelement 16 eines Implantats 18. Das Implantat 18 kann insbesondere in Form einer Knochenschraube 20 ausgebildet sein. In den Figuren 1 und 2 ist beispielhaft eine Knochenschraube 20 in Form einer Polyaxialschraube 22 schematisch dargestellt, die einen Schraubenschaft 24 umfasst, welcher von einem Längskanal 26 durchsetzt und mit einem Außengewinde 28 versehen ist. Ein distales Ende 30 des Schraubenschafts 24 ist offen und steht mit dem Längskanal 26 in Fluidverbindung. Optional können proximalseitig des Endes 30 noch ein oder mehrere seitliche Austrittsöffnungen 32 vorgesehen sein, die den Schaft quer zu seiner Längsachse 34 eröffnen. Die Austrittsöffnungen 32 stehen mit dem Längskanal 26 in Fluidverbindung.

Ein proximales Ende des Schraubenschafts 24 ist in Form eines kugeligen Kopfes 36 ausgebildet, auf dem eine Hülse 38 gelenkig gelagert ist. Die Hülse 38 dient insbesondere zur Aufnahme stab- oder plattenförmiger Verbindungselemente, die mit stabförmigen Abschnitten in eine Verbindungselementaufnahme 40 der Hülse 38 einlegbar sind. An der Hülse 38 ist ein Innengewinde 42 ausgebildet, in das ein in den Figuren nicht dargestelltes Fixierelement in Form einer Schraube einschraubbar ist, um das Verbindungselement in der Verbindungselementaufnahme 40 festzulegen.

Der Kopf 36 weist eine in proximaler Richtung weisend geöffnete Werkzeugaufnahme 44 auf, die in Form eines Innenmehrkant oder eines Innenvielrund ausgebildet ist. Optional kann die Werkzeugaufnahme 44 ferner mit einem Innengewinde versehen sein. Eine solche Werkzeugaufnahme 44 ist beispielsweise in der DE 20 2008 006 870 U1 beschrieben.

Das Instrument 10 umfasst einen hülsenförmigen Abschnitt 46, welcher
eine Längsachse 48 definiert. Im Inneren des Abschnitts 46 ist ein Knochenzementbehälter 50 angeordnet beziehungsweise ausgebildet, welcher eine erste Kammer 52 und eine zweite Kammer 54 umfasst. Dies erstrecken sich beide in Richtung der Längsachse 48 und sind durch ein Trennelement 56 in Form einer Trennwand 58 getrennt, die sich ebenfalls in Richtung der Längsachse 48 erstreckt. Distalseitig ist der hohlzylindrische Knochenzementbehälter 50 durch ein Verschlusselement 60 zu einem Knochenzementkanal 62 hin verschlossen.

Der Knochenzementkanal 62 durchsetzt einen Zementauslassstutzen, der sich distalseitig an den Knochenzementbehälter 50 anschließt und sich koaxial zur Längsachse 48 in distaler Richtung erstreckt. Ein distales Ende des Zementauslassstutzens bildet das erste Werkzeugelement 66, welches wahlweise als Außenmehrkant oder Außenvielrund oder mit einem Außengewinde versehen ausgebildet sein kann. Das erste Werkzeugelement 66 steht über ein Kupplungselement 16 vor. Dieses ist optional als Halteclip 68 ausgebildet, um das Instrument 10 klemmend im Bereich der Hülse 38 mit dieser zu koppeln. Der Halteclip 68 weist zwei
oder mehr in distaler Richtung weisende, den Zementauslassstutzen teilweise umgebende, in radialer Richtung federnde Vorsprünge 72 auf.

Das erste Werkzeugelement 66 kann entweder in das Innengewinde der Werkzeugelementaufnahme 44 eingeschraubt oder, wenn es als Aussenmehrkant oder Außenvielrund ausgebildet ist, in diese hineingesteckt werden. Mit dem Instrument 10 lässt sich ein Drehmoment auf den Kopf 36 übertragen, so dass die Knochenschraube 20 in einen Knochen 70, beispielsweise einen Pedikel eines Wirbels, eingeschraubt werden kann.

An einem proximalen Ende des Abschnitts 46 ist eine ringförmige Einschnürung 74 ausgebildet, die einen ringförmigen Vorsprung 76 distalseitig begrenzt. Die Einschnürung 74 und der Vorsprung 76 dienen zum Koppeln des Instruments 10 mit einer optionalen Vorschubeinrichtung 78. Die Vorschubeinrichtung 78 dient insbesondere zum Durchmischen und/oder Ausbringen von im Knochenzementbehälter 50 befindlichem Knochenzement 88. Insbesondere dient die Vorschubeinrichtung 78 zum Vorschieben des Knochenzements 88 in distaler Richtung.

Die Vorschubeinrichtung 78 umfasst insbesondere einen Stößel 80 mit einem kolbenförmigen Vorschubglied 82, welches ein distales Ende des Stößels 80 bildet. Ein proximales Ende des Stößels 80 bildet ein flacher scheibenförmiger Kopf 84, der als Betätigungselement 86 der Vorschubeinrichtung 78 dient. Er ist vorzugsweise unlösbar, insbesondere einstückig mit dem Stößel 80 ausgebildet.

Im Knochenzementbehälter 50 ist vorzugsweise bereits Knochenzement 88 enthalten, und zwar eine erste Komponente 90 in der ersten Kammer 52 und eine zweite Komponente 92 in der zweiten Kammer 54. Die beiden Komponenten 90 und 92, beispielsweise ein Binder und ein Härter, kommen miteinander in Kontakt und können miteinander reagieren, wenn das Trennelement 56 entfernt oder zerstört wird.

Zum Ausbringen des Knochenzements 88 mit der Vorschubeinrichtung 78 muss das Verschlusselement 60 entfernt oder zerstört werden. Dies kann insbesondere durch hinreichenden Druck erreicht werden, der mit der Vorschubeinrichtung 78 auf den Knochenzement 88 ausgeübt wird.

Die besondere Ausgestaltung des Instruments 10 ermöglicht es, direkt nach dem Einschrauben der Knochenschraube 20 in den Knochen 70 den Knochenzement 88 zu applizieren, ohne das Instrument 10 von der Knochenschraube 20 abkoppeln und einen separaten Applikator ankoppeln zu müssen. So kann vermieden werden, dass Knochenzement im Bereich der Hülse 38 austreten und diese verschmutzen kann, insbesondere im Bereich des Innengewindes 42 und der Verbindungselementaufnahme 40.

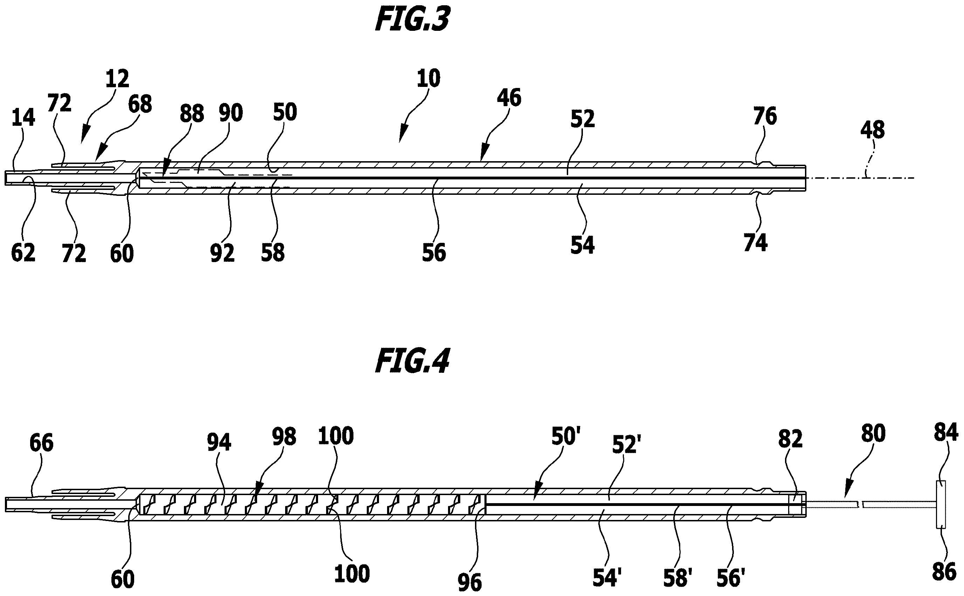

In Figur 4 ist beispielhaft ein weiteres Ausführungsbeispiel eines Instruments 10 mit einer alternativen Variante des Knochenzementbehälters 50' dargestellt. Dieser ist etwas kürzer als der Knochenzementbehälter 50. So umfasst der Abschnitt 46 nicht nur den Knochenzementbehälter 50', sondern auch einen Mischraum 94, der eingangs auch als Mischbereich bezeichnet wurde. Dieser erstreckt sich zwischen dem Knochenzementkanal 62 und dem Knochenzementbehälter 50', welcher durch ein weiteres, sich quer zur Längsachse 48 erstreckendes Verschlusselement 96 vom Mischraum 94 getrennt ist. Das Trennelement 56' ist in Form einer sich in Längsrichtung erstreckenden Trennwand 58' ausgebildet und trennt die erste Kammer 52' von der zweiten Kammer 54'.

Das Instrument 10 umfasst ferner eine Mischeinrichtung 98, die vorzugsweise im Mischraum 94 angeordnet ist. Sie umfasst eine Mehrzahl von Mischelementen 100, die in Form von Mischvorsprüngen ausgebildet sind, welche in Richtung auf die Längsachse 48 hin vorstehen. Sie sind relativ zur Längsachse 48 etwas geneigt und führen in Folge einer Vorschubbewegung des Stößels 80 zu einer Ablenkung des sich in distaler Richtung bewegenden Knochenzements 88 und zu einer verbesserten Durchmischung der beiden Komponenten 90 und 92 im Mischraum 94. Durch die Mischeinrichtung 98 und den Mischraum 94 kann eine optimale Vermischung der beiden Komponenten 90 und 92 des Knochenzements 88 und damit einer definierte Aushärtereaktion sichergestellt werden.

Beide beschriebenen Instrumente können ferner ein Griffelement 102 umfassen. Dieses kann insbesondere in Form einer herkömmlichen Kanüle ausgebil det sein und an seinem proximalen Ende zwei in radialer Richtung abstehende Flügel 104 umfassen, die ein Operateur mit Zeigefinger und Mittelfinger zur Abstützung nutzen kann, um mit dem Daumen das Betätigungselement 86 in distaler Richtung vorzuschieben und den Knochenzement 88 zu applizieren beziehungsweise vor dem Applizieren durch den Mischraum 94 zu drücken.

Das Instrument 10 ist vorzugsweise einstückig aus einem Kunststoff ausgebildet, der sterilisierbar ist. Das Instrument 10 kann optional auch aus einem Metall hergestellt sein.

Das Instrument 10 ermöglicht es, die Funktionen eines Schraubendrehers, eines Applikators, eines Mischers und eines Speichers für den Knochenzement 88 beziehungsweise dessen Komponenten 90 und 92 zu vereinen. So kann die Handhabung des Knochenzements 88 vereinfacht und gleichzeitig die Gefahr eines unerwünschten Knochenzementaustritts verhindert werden.

Der Ablauf der Anwendung des Instruments 10 sowie der Implantation des Implantats 18 kann beispielsweise wie folgt erfolgen:
Zunächst wird das Instrument 10 steril vorbereitet. Die Knochenschraube 20 wird mit dem Instrument 10 gekoppelt und anschließend am Knochen 70 platziert und eingeschraubt.

In einem nächsten Schritt wird der Knochenzement 88 gemischt, beispielsweise durch Herausbrechen des Trennelements 56 oder durch Einpressen der beiden Komponenten 90 und 92 in den Mischraum 94. Als nächstes wird abgewartet, bis der Knochenzement 88 die für die Verarbeitung erforderliche Viskosität aufweist. Sobald er diese erreicht hat, wird er mit dem Stößel 80 durch den Knochenzementkanal 62 in den Längskanal 26 appliziert.

Nun kann die Aushärtezeit abgewartet werden. Sobald der Knochenzement 88 hinreichend fest ist, kann zuletzt das Instrument 10 entfernt werden, beispielsweise durch Abziehen oder Abschrauben, je nachdem wie das Instrument 10 mit der Knochenschraube 20 gekoppelt wurde.

Wie bereits erwähnt, wird die Kopplung zwischen dem Instrument 10 und dem Implantat 18 vorzugsweise in einer Form realisiert, wie sie in der DE 20 2008 006 870 beschrieben ist. Mit anderen Worten wird das Instrument 10 vorzugsweise zementdicht, jedoch luftdurchlässig an das Implantat 18 gekoppelt. Dies ermöglicht es, das Instrument 10 in seiner Funktion als Applikator zu entlüften, so dass nicht übermäßig viel Luft in den Knochen 70 eingepresst wird.

Die Größe des Knochenzementbehälters 50 ist vorzugsweise an das mit dem Instrument zu implantierende Implantat 18 angepasst, so dass genau die richtige Menge des Knochenzements 88 für das Implantat 18 bereitgestellt wird. Mit dem Instrument 10 ist kein externes Mischen mehr erforderlich, so dass weder eine Verschmutzung beziehungsweise eine Kontamination des Wundbereichs noch das Risiko für eine Verschmutzung der Knochenschraube 20 besteht.

Das Instrument 10 kann nach Gebrauch einfach entsorgt werden. Daher ist es vorzugsweise als Einweginstrument ausgebildet.

## Patentansprüche

1. Medizinisches Instrument (10) umfassend einen Werkzeugabschnitt (12) mit einem ersten Werkzeugelement (66) zum in Eingriff Bringen mit einem korrespondierenden zweiten Werkzeugelement (16) an einem Implantat (18) und mit einem am oder im Instrument (10) angeordneten oder ausgebildeten Knochenzementbehälter (50), **dadurch gekennzeichnet, dass** das Instrument (10) insgesamt einstückig ausgebildet ist.

2. Medizinisches Instrument nach Anspruch 1, **gekennzeichnet durch** mindestens einen hülsenförmigen Abschnitt (46), wobei der Knochenzementbehälter (50) im Bereich des hülsenförmigen Abschnitts (46) angeordnet oder ausgebildet ist.

3. Medizinisches Instrument nach einem der voranstehenden Ansprüche, **dadurch gekennzeichnet, dass** der Knochenzementbehälter (50) mindestens eine erste Kammer (52) und mindestens eine zweite Kammer (54) umfasst und dass die mindestens eine erste und die mindestens eine zweite Kammer (52, 54) durch mindestens ein Trennelement (56) räumlich voneinander getrennt sind.

4. Medizinisches Instrument nach Anspruch 3, **dadurch gekennzeichnet, dass** das mindestens eine Trennelement (56) in Form einer Trennwand (58) ausgebildet ist, die sich quer oder parallel zu einer Längsachse (48) des Knochenzementbehälters (50) erstreckt.

5. Medizinisches Instrument nach einem der voranstehenden Ansprüche, **gekennzeichnet durch** eine Vorschubeinrichtung (78) zum Durchmischen und/oder Ausbringen von im Knochenzementbehälter (50) befindlichen Knochenzement (88), insbesondere zum Vorschieben des Knochenzements (88) in distaler Richtung.

6. Medizinisches Instrument nach einem der voranstehenden Ansprüche, **dadurch gekennzeichnet, dass** das erste Werkzeugelement (66) einen Knochenzementkanal (62) umfasst, welcher das erste Werkzeugelement (66) durchsetzt und mit dem Knochenzementbehälter (50) in Fluidverbindung steht.

7. Medizinisches Instrument nach Anspruch 6, **gekennzeichnet durch** ein Verschlusselement (60; 96), welches den Knochenzementkanal (62) und/oder den Knochenzementbehälter (50) zum Knochenzementkanal (62) hin verschließt.

8. Medizinisches Instrument nach einem der voranstehenden Ansprüche, **gekennzeichnet durch** einen Zementauslassstutzen, welcher distalseitig über das erste Werkzeugelement (66) vorstehend ausgebildet ist.

9. Medizinisches Instrument nach einem der voranstehenden Ansprüche, **dadurch gekennzeichnet, dass** der Knochenzementbehälter (50) mindestens einen Mischbereich (94) umfasst, welcher eine Mehrzahl von Mischelementen (100) umfasst.

10. Medizinisches Instrument nach einem der voranstehenden Ansprüche, **dadurch gekennzeichnet, dass** der Knochenzementbehälter (50) Knochenzement (88) enthält.

11. Medizinisches Instrument nach Anspruch 10, **dadurch gekennzeichnet, dass** der Knochenzement (88) mindestens eine erste Knochenzementkomponente (90) und eine zweite Knochenzementkomponente (92) umfasst, die räumlich voneinander getrennt im Knochenzementbehälter (50) enthalten sind.

12. Medizinisches Instrument nach einem der voranstehenden Ansprüche, **dadurch gekennzeichnet, dass** das erste Werkzeugelement (66) in Form eines Werkzeugvorsprungs ausgebildet ist zum in Eingriff Bringen mit einer korrespondierend ausgebildeten Werkzeugaufnahme (44) an einem Implantat (18).

13. Medizinisches Instrument nach einem der voranstehenden Ansprüche, **dadurch gekennzeichnet, dass** das erste Werkzeugelement (66) in Form eines Mehrkants, eines Vielrunds oder eines Gewindeabschnitts ausgebildet ist.

14. Medizinisches Instrument nach einem der voranstehenden Ansprüche, **dadurch gekennzeichnet, dass** das Instrument (10) durch Spritzgießen ausgebildet ist.

15. Medizinisches Instrument nach einem der voranstehenden Ansprüche, **dadurch gekennzeichnet, dass** das Instrument (10) in Form eines Schraubendrehers ausgebildet ist.

## Claims

1. Medical instrument (10) comprising a tool portion (12) with a first tool element (66) for engagement with a corresponding second tool element (16) on an implant (18), further comprising a bone cement container (50) arranged or formed on or in the instrument (10), **characterized in that** the instrument (10) is formed entirely in one piece.

2. Medical instrument according to claim 1, **characterized by** at least one portion (46) in the form of a sleeve, wherein the bone cement container (50) is arranged or formed in the region of the portion (46) in the form of a sleeve.

3. Medical instrument according to any one of the preceding claims, **characterized in that** the bone cement container (50) comprises at least one first compartment (52) and at least one second compartment (54) and **in that** the at least one first and the at least one second compartment (52, 54) are separated from one another spatially by at least one separator element (56).

4. Medical instrument according to claim 3, **characterized in that** the at least one separator element (56) takes the form of a partition (58), which extends transversely of or parallel to a longitudinal axis (48) of the bone cement container (50).

5. Medical instrument according to any one of the preceding claims, **characterized by** a feed device (78) for intermixing and/or discharge of bone cement (88) located in the bone cement container (50), in particular for feeding the bone cement (88) in the distal direction.

6. Medical instrument according to any one of the preceding claims, **characterized in that** the first tool element (66) comprises a bone cement channel (62), which passes through the first tool element (66) and is in fluid connection with the bone cement container (50).

7. Medical instrument according to claim 6, **characterized by** a closing element (60; 96) which closes the bone cement channel (62) and/or the bone cement container (50) relative to the bone cement channel (62).

8. Medical instrument according to any one of the preceding claims, **characterized by** a cement outlet nozzle, which is formed to protrude beyond the first tool element (66) at the distal end.

9. Medical instrument according to any one of the preceding claims, **characterized in that** the bone cement container (50) comprises at least one mixing region (94), which comprises a plurality of mixing elements (100).

10. Medical instrument according to any one of the preceding claims, **characterized in that** the bone cement container (50) contains bone cement (88).

11. Medical instrument according to claim 10, **characterized in that** the bone cement (88) comprises at least one first bone cement component (90) and one second bone cement component (92), which are contained in spatially separated manner in the bone cement container (50).

12. Medical instrument according to any one of the preceding claims, **characterized in that** the first tool element (66) takes the form of a tool projection for engagement with a correspondingly formed tool receptacle (44) on an implant (18).

13. Medical instrument according to any one of the preceding claims, **characterized in that** the first tool element (66) takes the form of a polygon, a multiple lobe member or a threaded portion.

14. Medical instrument according to any one of the preceding claims, **characterized in that** the instrument (10) is formed by injection moulding.

15. Medical instrument according to any one of the preceding claims, **characterized in that** the instrument (10) takes the form of a screwdriver.

## Revendications

1. Instrument médical (10) comprenant une partie d'outil (12) présentant un premier élément d'outil (66) destiné à entrer en contact avec un deuxième élément d'outil (16) correspondant sur un implant (18), et comprenant un contenant à ciment à os (50) agencé ou formé sur ou dans l'instrument (10), **caractérisé en ce que** l'instrument (10) est réalisé dans l'ensemble d'une seule pièce.

2. Instrument médical selon la revendication 1, **caractérisé par** au moins une partie en forme de manchon (46), dans lequel le contenant à ciment à os (50) est agencé ou formé dans la zone de la partie en forme de manchon (46).

3. Instrument médical selon l'une quelconque des revendications précédentes, **caractérisé en ce que** le contenant à ciment à os (50) comprend au moins une première chambre (52) et au moins une deuxième chambre (54) et **en ce que** ladite au moins une première chambre et ladite au moins une deuxième chambre (52, 54) sont séparées l'une de l'autre dans l'espace par au moins un élément de séparation (56).

4. Instrument médical selon la revendication 3, **caractérisé en ce que** ledit au moins un élément de séparation (56) est réalisé sous la forme d'une paroi de séparation (58) qui s'étend transversalement ou parallèlement à un axe longitudinal (48) du contenant à ciment à os (50).

5. Instrument médical selon l'une quelconque des revendications précédentes, **caractérisé par** un dispositif d'avancement (78) pour mélanger et/ou distribuer le ciment à os (88) situé dans le contenant à ciment à os (50), en particulier pour faire avancer le ciment à os (88) dans le sens distal.

6. Instrument médical selon l'une quelconque des revendications précédentes, **caractérisé en ce que** le premier élément d'outil (66) comprend un canal de ciment à os (62) qui traverse le premier élément d'outil (66) et qui est en liaison fluidique avec le contenant à ciment à os (50).

7. Instrument médical selon la revendication 6, **caractérisé par** un élément de fermeture (60 ; 96) qui ferme le canal de ciment à os (62) et/ou le contenant à ciment à os (50) en direction du canal de ciment à os (62).

8. Instrument médical selon l'une quelconque des revendications précédentes, **caractérisé par** une tubulure de sortie de ciment, laquelle fait saillie du côté distal du premier élément d'outil (66).

9. Instrument médical selon l'une quelconque des revendications précédentes, **caractérisé en ce que** le contenant à ciment à os (50) comprend au moins une zone de mélange (94), laquelle comporte une pluralité d'éléments de mélange (100).

10. Instrument médical selon l'une quelconque des revendications précédentes, **caractérisé en ce que** le contenant à ciment à os (50) contient du ciment à os (88).

11. Instrument médical selon la revendication 10, **caractérisé en ce que** le ciment à os (88) comprend au moins un premier composant de ciment à os (90) et un deuxième composant de ciment à os (92), qui sont contenus dans le contenant à ciment à os (50) en étant séparés dans l'espace l'un de l'autre.

12. Instrument médical selon l'une quelconque des revendications précédentes, **caractérisé en ce que** le premier élément d'outil (66) est réalisé sous la forme d'une partie saillante d'outil destinée à entrer en contact avec un logement d'outil (44) réalisé de manière correspondante sur un implant (18).

13. Instrument médical selon l'une quelconque des revendications précédentes, **caractérisé en ce que** le premier élément d'outil (66) est réalisé sous la forme d'un embout à pans multiples, d'un embout à arrondis multiples ou d'une partie filetée.

14. Instrument médical selon l'une quelconque des revendications précédentes, **caractérisé en ce que** l'instrument (10) est conçu par moulage par injection.

15. Instrument médical selon l'une quelconque des revendications précédentes, **caractérisé en ce que** l'instrument (10) est réalisé sous la forme d'un tournevis.
